# EUROPEAN PATENT APPLICATION

(11) **EP 2 674 416 A1**
(43) Date of publication of application: **18.12.2013**
(21) Application number: 12171520.5
(22) Date of filing: 11.06.2012
(51) Int. Cl.: C07C 253/04, C07C 255/09

(54) **Process for the preparation of alkali metal and alkaline earth metal tricyanomethanides**

(71) Applicant: Lonza Ltd, 3930 Visp (CH)
(72) Inventor: The designation of the inventor has not yet been filed

(57) **Abstract**

The present invention relates to a safe industrially applicable process for preparing alkali metal and alkaline earth metal tricyanomethanides.

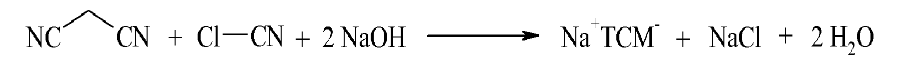

## Description

The present invention relates to a process for preparing alkali metal and alkaline earth metal tricyanomethanides as starting material for the preparation of ionic liquids according to the following equation for sodium tricyanomethanide (NaTCM).

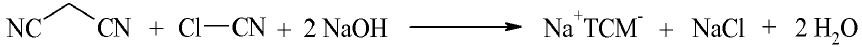

Ionic liquids made from alkali metal or alkaline earth metal tricyanomethanides are important raw materials and auxiliaries in the electronics industry for, inter alia, the production of rechargeable batteries.

Various processes for preparing tricyanomethanides are known. The cyanidation of malononitrile was described for the first time by Schmidtmann in Chem. Ber. 1896, 29, 1168-1175. In the process malononitrile was deprotonated by means of sodium ethoxide in ethanol and subsequently converted into sodium tricyanomethanide by stepwise addition of cyanogen chloride and subsequently crystallized from ether. In this process, sodium tricyanomethanide was isolated in a yield of about 70%.

In Chem. Ber. 1929, 62B, 153-163, Birckenbach et al. described the cyanidation of malononitrile by means of cyanogen bromide. Birckenbach et al. and Mayer et al. (Monatsh. Chem., 1969, 100, 462) have described the preparation of silver tricyanomethanide which has a low halogen content by admixing crude alkali metal tricyanomethanide with silver nitrate, resulting in an initial precipitation of silver chloride or silver bromide. Addition of further silver nitrate to the filtrate enables silver tricyanomethanide to be isolated. In addition, Mayer et al. have described the reaction of silver tricyanomethanide with cyanogen chloride at 100 °C for 40 h to form tetracyanomethane which was sublimed and subsequently hydro lysed in sulfuric acid to form ammonium tricyanomethanide. Lithium tricyanomethanide was obtained by Mayer by addition of lithium chloride to an acetonitrile solution of tetracyanomethane at -96°C.

A further process has been disclosed by Trofimenko et al. in J. Org. Chem. 1962, 27, 433, in which potassium tricyanomethanide was obtained by treating a dihalomalononitrile-potassium bromide complex with potassium cyanide.

In Bull. Soc. Chim. Fr. 1954, 948, Cox et al. have described a further process for preparing of potassium tricyanomethanide at low temperature, in which bromomalononitrile was reacted with potassium cyanide.

Further processes for preparing tricyanomethanides comprising the reaction of deprotonated malononitrile with phenyl cyanate have been disclosed by Grigat et al. in Chem. Ber. 1965, 98, 3777-3784, and Martin et al. in DD-A-48614. Yields of from 75 to 88% were obtained here.

In WO-A-2008/019852 a process for preparing alkali metal and alkaline earth metal tricyanomethanides is disclosed. Although this process can be applied to a large variety of tricyanomethanides, the process still comprises problems in large scale production in view of side products, malononitrile stability and the danger of cyanogen halide loss during an accident. In this process it is essential to control the pH within the range of 4 to 9.5, which is difficult to perform at industrial scale. Another unsolved problem of the process according to WO-A-2008/019852 is the deposition of salts and crusts, mainly consisting of an alkali metal or alkaline earth metal hydroxide or halide. These depositions on the reactor wall can result in stirring problems and thus render the process critical for large scale production. A blocked stirrer would cause uncontrollable temperature rise with release of cyanogen chloride onto the atmosphere.

In WO-A- 2009/021751 a process for preparing alkali metal and alkaline earth metal tricyanomethanides having high purity is disclosed.

However the process still comprises problems in large scale production in view of safety. In this process there was an excess of base resulting in a pH in the range of 13 to 14 at any time during the reaction. Under these conditions it is also not possible to obtain a homogeneous phase. In the reaction vessel precipitation occurred, which is not compatible with industrial scale production. Thus the process also results in uncontrollable precipitation of crusts and the problems outlined above.

Therefore, the object of the instant invention was to provide a safe process suitable for industrial production.

This object was achieved as set forth in the present invention.

Provided is a process for the preparation of an alkali metal or alkaline earth metal tricyanomethanide, said process comprising a triple parallel dosage of (i) malononitrile, (ii) a cyanogen halide and (iii) an alkali metal base or alkaline earth metal base in the presence of a solvent. The pH of the reaction mixture has an impact in the formation of secondary products, such as compounds of formula 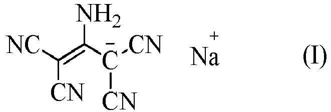 and 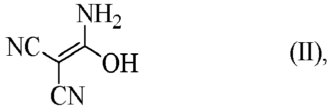 which are formed at high pH values when using sodium hydroxide as base. Therefore and in order to be sure that no excess of base is present in the reaction mixture a pre-addition of 5 wt-% of the whole added amount of malonitrile is preferred. Expediently, a suitable additional amount of the cyanogen halide is dosed after the triple parallel dosage to ensure that no unreacted malononitrile remains in the reaction mixture. Preferably, after the triple parallel dosage has finished the pH of the resulting reaction mixture is pH 10.0 or lower. Preferably, the pH is in a range of from pH 10.0 to 8.0. Also expediently, the additional cyanogen halide is used to adjust to pH to the preferred range.

Exothermicity tests for the process described in WO-A-2009/021751 (process A) and for the triple dosage herein disclosed (triple dosage process) have been performed. By dynamic DSC (Differential Scanning Calorimetry) (4 K/min, 30 to 300 °C) several exothermicities were detected above 136 °C and the total exothermicity was -635 J/g for the process A. For the triple dosage process several exothermicities were detected above 210 °C and the total exothermicity was -268 J/g. The exothermicity test results clearly show that the process herein disclosed is much safer than the one disclosed in WO-A-2009/021751.

Triple parallel dosage as proposed in the present invention maintains a low concentration of free malononitrile in the reaction mixture and thus avoids enrichment of unreacted malononitrile. Controlling malononitrile at a low concentration dramatically diminishes the danger of explosion. Malononitrile stability depends on pH, temperature and time. Spontaneous decomposition/polymerization reactions can occur above 100 °C. Explosions can occur in basic medium (pH > 8) even at temperatures below 100 °C. On the other hand malononitrile is also explosive at acidic pH (i.e. pH < 4) at temperatures above 130 °C.

Expediently, during the triple addition the temperature of the reaction mixture is controlled to 20 °C or lower, preferably at a temperature of 15 °C or lower, most preferably between 0 and 15 °C. The dosage time is not a critical factor for the reaction as long as the temperature does not exceed a temperature of approx. 25 °C during or after the addition.

The triple dosage can be carried out discontinuously, preferably in small portions, or continuously, whereas carrying out the triple dosage continuously is preferred. Both modes, continuous and discontinuous mode, may be mixed to a certain extent. Optimal temperature control can be performed using continuous triple dosage.

As the triple dosage process is safer and the addition of reagents is temperature controlled it has been proved that the reaction times of process herein disclosed when compared to the previously described in WO-A-2009/021751 are shorter compared to the process described in WO-A-2009/021751 and that the shorter reaction times do decrease neither yield nor purity of the final product.

An advantage of the present invention is the gain in safety during the reaction, especially in view of large scale production. According to the present invention during the triple addition the pH of the reaction is always below 12, where the cyanidation mixture is most stable. Almost no precipitation could be observed even at large scale production. Compared to WO-A-2009/021751 the product quality is increased and fewer impurities are formed.

Since ionic liquids can only be purified with difficulty the improved process leading to highly pure tricyanomethanides has economic advantages, as well. Thus, the quality of the product is achieved without introducing additional unit operations.

Furthermore, the triple dosage has the advantage that the emulsion formed is stable and stirrable until the end of the addition of all reagents, which increases safety.

Neither phase separation no precipitation occurs at laboratory or production scale.

The triple dosage also allows carrying out the process in a continuous mode with subsequent separation of the salt from the product. Preferably in continuous mode the reaction can be carried out in a tubular reactor, more preferably in a microreactor.

In the instant triple dosage process, even at smaller scale, a 26% increase of yield of the raw product could be reached compared to the process according to WO-A- 2009/021751. Thus, the increase in safety and better handling in addition also provides a significant commercial advantage.

The present process limits the risk of danger of formation of explosives in the reaction mixture.

The product obtained in the instant process contains a lower concentration of by-products than in the case of a reaction in water. Compared to a reaction solely in water, the reaction in a solvent comprising at least one organic solvent and water has the advantage that the pH of the reaction mixture does not have to be kept within a constant and narrow range, the pH in the present triple dosage process is essentially self- adjusted. It is only necessary to ensure sufficient deprotonation of the malononitrile at the beginning of the triple dosage. The amount of base necessary for this can be determined in a known way from the pKₐ of the compounds used.

In the process of the invention, the alkali metal or alkaline earth metal tricyanomethanide precipitates preferably in the form of coarse particles.

In a preferred embodiment during the triple parallel dosage malononitrile, the cyanogen halide and the alkali metal base or alkaline earth metal base are fed in a molar ratio in the range from 0.9:1:2 to 1:2:3.

The malononitrile is particularly preferably used in a molar ratio to cyanogen halide from 0.9:1 to 1:2, preferably from 1:1 to 1:1.5. A slight excess of cyanogen halide after completion of the reaction is preferred. Expediently, the molar ratio of cyanogen halide to malononitrile is between 1:1 to 1:1.2.

The addition rate of the different reagents is not pH controlled but temperature controlled.

The malononitrile is preferably used in a molar ratio to the alkali metal base or alkaline earth metal base of from 1:2 to 1:3, preferably from 1:2 to 1:2.4, more preferably of from 1:2 to 1:2.2 and most preferably approx. 1:2.06.

As cyanogen halide, it is possible to use cyanogen chloride or cyanogen bromide.

Preference is given to use cyanogen chloride as cyanogen halide.

Furthermore, the alkali metal or alkaline earth metal base is particularly preferably a strong base. In particular, said alkali metal or alkaline earth metal base can be selected from the group consisting of alkali metal or alkaline earth metal hydroxides and alkali metal or alkaline earth metal oxides, alkali metal or alkaline earth metal alkoxides. As alkali metal or alkaline earth metal alkoxides, particular preference is given to using respective C₁₋₆-alkoxides. Suitable alkali metal or alkaline earth metal C₁₋₆-alkoxides are, for example, sodium or potassium salts of C₁₋₆-alcohols such as methanol, ethanol, isopropanol, *n*-butanol, *sec*-butanol, *tert*-butanol, pentanol and hexanol.

In a preferred process variant, the alkali metal or alkaline earth metal base is a lithium, sodium, potassium, calcium, magnesium or barium base.

Expediently the base is used as an aqueous mixture, preferably an aqueous solution.

In a preferred embodiment the alkali metal base is a sodium and/or potassium base. In a more preferred embodiment the base is sodium hydroxide.

The concentration of the base is in the range of 25 to 40 wt-%, preferably in the range of 30 to 40 wt-% and more preferably in the range of 35 to 38 wt-%.

The solvent in the reaction mixture comprises water and at least one organic solvent selected from the group consisting of ethers, alcohols, ketones, amides and organic nitriles.

Particularly preferred organic solvents are, for example 2-propanol, *sec*-butanol, pentanol, ethylene glycol, diethyleneglycol, triethylenglycol, *tert*-butanol, isoamyl alcohol, 1,2-propanediol, 1,3-propanediol, 1,4-butanediol, benzyl alcohol, 2-pentanol, 1-butanol, 1-propanol, 2-methoxyethanol, cyclohexanol, glycerol, 2-ethyl hexanol, acetone, cyclopentanone, cyclohexanone, 2-pentanone, 3-pentanone, methyl ethyl ketone, methyl isobutyl ketone, methyl *tert*-butyl ether, diethyl ether, diisopropyl ether, tetrahydrofurane, 2-methyltetrahydrofuran, dioxane, diglyme, ethylene glycol diethyl ether and ethylene glycol dimethyl ether, diphenyl ether, cyclopentyl methyl ether, anisol, ethoxybenzene, dimethylacetamide, *N*-methylformamide, formamide, dimethylformamide, acetonitrile, propionitrile, butyronitrile, 2-methylbutyronitrile, valeronitrile and/or mixtures thereof.

In a preferred process variant, the aqueous solvent contains a nitrile, particularly preferably acetonitrile.

Preferably the organic solvent is at least partially miscible with water.

Optionally not more than 10 wt-%, preferably about 4 to 6 wt-%, of the whole added amount of malononitrile is present in the vessel prior to the start of the triple parallel dosage.

In a preferred process variant, in the second step the obtained alkali metal or alkaline earth metal tricyanomethanide is dissolved, if appropriate at elevated temperature, in acetone, methyl ethyl ketone and/or methyl isobutyl ketone and, then again if appropriate at a reduced temperature, precipitated. It has here been found to be advantageous for the alkali metal tricyanomethanide solution also to comprise activated carbon. The activated carbon can be filtered off, optionally at elevated temperature, before the alkali metal or alkaline earth metal tricyanomethanide is precipitated.

Precipitation of the alkali metal or alkaline earth metal tricyanomethanide is, if appropriate after removal of the activated carbon, can be carried out in the presence of methyl *tert*-butyl ether or diisopropyl ether. The alkali metal or alkaline earth metal tricyanomethanide is preferably precipitated at a temperature below 30 °C, particularly preferably at a temperature at or below 20°C.

Preferably the alkali metal or alkaline earth metal tricyanomethanide is obtained in a purity of at least 99.5%, particularly preferably in a purity of at least 99.8%, in the second step. According to the present invention alkali metal or alkaline earth metal tricyanomethanides can be directly obtained in a purity of 99.9% and higher without further purification operations. In the examples, sodium tricyanomethanide, for example, having a halide content of less than 10 ppm was obtained.

### Examples:

In the examples described below, replacement of the sodium hydroxide by another alkali metal or alkaline earth metal base leads to the corresponding alkali metal or alkaline earth metal tricyanomethanide. The use of mixtures of bases of different alkali metals or alkaline earth metals also enables mixed alkali metal or alkaline earth metal tricyanomethanides to be obtained. The use of mixtures of bases comprising the same alkali metals or alkaline earth metals leads to one single alkali metal or alkaline earth metal tricyanomethanides.

### Example 1:

Acetonitrile (145.75 g, 3.55 mol, 4.7 eq) and malononitrile (4.9 g of a 50 wt-% solution in acetonitrile, 37.1 mmol) were added to a vessel. Temperature was set at 20°C under stirring.

A simultaneous dosage of malononitrile (95 g of a 50 wt-% solution in acetonitrile, 0.72 mol), sodium hydroxide (179.6 g of a 34.7 wt-% aqueous solution, 1.56 mol, 2.06 eq) and cyanogen chloride (46.5 g, 0.76 mol, 1eq) was done. The temperature during the triple dosage was kept at 15 °C and the dosage times were of 105 min for the malononitrile, 120 min for the sodium hydroxide and 120 min for the cyanogen chloride. After the triple addition the reaction mixture was stirred at 15 °C for 10 min. Additional cyanogen chloride (2.9 g, 0.05 mol, 0.06 eq) was added. After stirring the reaction mixture at 50°C for 30 min the pH measured was 9.41 (pH <10). The resulting suspension was filtered off. The mother liquor (414.4 g) containing sodium tricyanomethanide was analyzed by NMR and titration and the assay measured was 19.8 wt-% for NaTCM. The crude yield obtained was 96%. After acetonitrile/- water (200 mbar, 45 °C) had been distilled off partly (174 g), the remaining reaction mixture was cooled from 45 °C to -5 °C during 3 h. The precipitated NaTCM was filtered off and washed twice with pre-cooled acetonitrile (2×20 g). Solid NaTCM (50 g, 440 mmol, 58% yield at >99% purity) was obtained. Without recrystallization the coarsely particulate product was colorless and contained less than 0.5% by weight of by-products and <1000 ppm of chloride.

### Example 2:

Acetonitrile (454.1 kg, 10.81 kmol, 4.7 eq) was placed in a vessel reactor and an initial portion of malononitrile (15.6 kg of 50 wt-% solution in acetonitril, 0.12 kmol) was added while the temperature in the reactor was controlled to be about 15 °C. After completing initial dosing, malononitrile (296.1 kg of 50 wt-% solution in acetonitrile, 2.24 kmol), NaOH (518.3 kg of 37.5 wt-% solution in water, 4.86 kmol, 2.06 eq) and cyanogen chloride (146.4 kg, 2.38 kmol, 1 eq, gaseous) were simultaneously added, keeping the temperature at 15 °C. During the reaction the temperature was controlled to be about 15 ± 1 °C. Cyanogen chloride was added via dip tube, wherein the exit of the plunger was always below the surface of the reaction mixture. During the reaction the pH was between pH 9 to 12. Finally a slight excess of cyanogen chloride (4.4 kg, 0.07 kmol, gaseous, approx. 0.03 eq) was added. The pH measured was about 9 to 10. The reaction mixture was heated to 50 °C and stirred at that temperature for additional 30 min. During the whole reaction no deposition of salts or crusts could be observed at the walls of the reactor. The reaction mixture remained a stirrable emulsion during the inventive triple dosage. Precipitated salt (NaCl) was removed by centrifugation at about 15 to 20 °C. Obtained was a clear orange-brown sodium tricyanomethanide (NaTCM) solution (crude yield: 99%).

### Example 3:

Acetonitrile (21.2 kg, 0.52 kmol, 4.70 eq) and malononitrile (725 g of a 50 wt-% solution in acetonitrile, 5.5 mol) were charged in a 70 L reactor and the temperature was set at 15°C under stirring. A simultaneous dosage of malonotrile (13.78 kg of a 50 wt-% solution in acetonitrile, 0.10 kmol), sodium hydroxide (26.1 kg of a 34.6 wt-% aqueous solution, 0.23 kmol, 2.06 eq) and cyanogen chloride (6.9 kg, 0.11 kmol, 1.02 eq) was done. The temperature during the triple dosage was kept in the range of 10 to 15 °C and the dosage times were 390 min for the malononitrile, 410 min for the sodium hydroxide and 440 min for the cyanogen chloride. After the triple addition the reaction mixture was stirred at 15 °C for 20 min. The pH measured was 12.4. Additional cyanogen chloride (60 g, 0.98 mol, 0.01 eq) was added. After stirring the reaction mixture at 50 °C for 30 min the pH measured was between 9 and 10. The mixture was cooled to 20°C and the resulting suspension was filtered off. The mother liquor (62.48 kg) containing sodium tricyanomethanide was analyzed by NMR and titration and the assay measured was 20 wt-% for NaTCM. The crude yield obtained was quantitative.

### Comparative Example 1:

Acetonitrile (27.1 kg, 0.660 mol, 6 eq), sodium hydroxide (18.1 kg of a 50 wt-% aqueous solution, 0.227 mol, 1.06 eq) and water (2.9 kg, 0.161 mol, 1.5 eq) were charged in a reactor vessel and cooled to 15 °C under stirring. Malononitrile (2.8 kg of a 85 wt-% solution in methanol, 0.036 mol) was added. A simultaneous dosage of malonotrile (5.66 kg of a 85 wt-% solution in methanol, 0.073 mol) and cyanogen chloride (7 kg, 0.114 mol, 1.04 eq) was done. The temperature during the double dosage was kept at 15 °C and the dosage times were of 4 h for the malononitrile, and 6 h for the cyanogen chloride. During the double addition the reaction mixture became a suspension and deposition of solid material in the walls of the reactor was observed. The pH during the first 4 h of addition of malononitrile and cyanogen chloride was 14. Once the whole amount of malononitrile was added and the dosage of cyanogen chloride was continued the pH measured was 8.3. The mixture was cooled to 10 °C and the suspension was filtered off. The mother liquor (54.77 kg) containing sodium tricyanomethanide was analyzed by NMR and the assay measured was 16 wt-% for NaTCM. The crude yield obtained was 71 %.

## Claims

1. A process for the preparation of an alkali metal or alkaline earth metal tricyanomethanide, said process comprising a triple parallel dosage of (i) malononitrile, (ii) a cyanogen halide and (iii) an alkali metal or alkaline earth metal base in the presence of a solvent.

2. The process according to claim 1, wherein during the triple parallel dosage malononitrile, the cyanogen halide and the alkali metal base or alkaline earth metal base are fed in a molar ratio in the range from 0.9:1:2 to 1:2:3.

3. The process according to claims 1 or 2, **characterized in that** the malononitrile is used in a molar ratio to cyanogen halide from 0.9:1 to 1:2, preferably from 1:1 to 1:1.5.

4. The process according to any of claims 1 to 3, **characterized in that** the molar ratio of malononitrile to the alkali metal or alkaline earth metal base is in the range of 1:2 to 1:3, preferably 1:2 to 1:2.4.

5. The process according to claims 1 to 4, **characterized in that** the cyanogen halide is cyanogen chloride or cyanogen bromide.

6. The process according to any of claims 1 to 5, **characterized in that** the alkali metal or alkaline earth metal base selected from the group consisting of alkali metal or alkaline earth metal hydroxides, alkali metal or alkaline earth metal oxides and alkali metal or alkaline earth metal alkoxides.

7. The process according to any of claims 1 to 6, **characterized in that** the alkali metal or alkaline earth metal base is selected from the group consisting of lithium, sodium, potassium, calcium, magnesium and barium bases.

8. The process according to any of claims 1 to 7, **characterized in that** the base is sodium hydroxide and/or potassium hydroxide.

9. The process according to any of claims 1 to 8, **characterized in that** the concentration of the base is in the range of 25 to 40 wt-%, preferably in the range of 30 to 40 wt-%.

10. The process according to any of claims 1 to 9, **characterized in that** the solvent comprises water and at least one organic solvent selected from the group consisting of ethers, alcohols, ketones, amides and organic nitriles.

11. The process according to claim 10, **characterized in that** the organic solvent is selected from the group consisting of 2-propanol, *sec*-butanol, pentanol, ethylene glycol, diethyleneglycol, triethylenglycol, *tert*-butanol, isoamyl alcohol, 1,2-propanediol, 1,3-propanediol, 1,4-butanediol, benzyl alcohol, 2-pentanol, 1-butanol, 1-propanol, 2-methoxyethanol, cyclohexanol, glycerol, 2-ethyl hexanol, acetone, cyclopentanone, cyclohexanone, 2-pentanone, 3-pentanone, methyl ethyl ketone, methyl isobutyl ketone, methyl *tert*-butyl ether, diethyl ether, diisopropyl ether, tetrahydrofurane, 2-methyltetrahydrofuran, dioxane, diglyme, ethylene glycol diethyl ether and ethylene glycol dimethyl ether, diphenyl ether, cyclopentyl methyl ether, anisol, ethoxybenzene, dimethylacetamide, *N*-methylformamide, formamide, dimethylformamide, acetonitrile, propionitrile, butyronitrile, 2-methylbutyronitrile, valeronitrile and mixtures thereof.

12. The process according to any of claims 1 to 11, wherein not more than 10 wt-%, preferably about 4 to 6 wt-%, of the whole added amount of malononitrile is present in the vessel prior to the start of the triple parallel dosage.
